Europäisches Patentamt

European Patent Office

Office européen des brevets

(19)

(11) Veröffentlichungsnummer: **0 019 817**
**A1**

# EUROPÄISCHE PATENTANMELDUNG

(21) Anmeldenummer: **80102735.0**

(22) Anmeldetag: **16.05.80**

(51) Int. Cl.³: **A 61 M 5/14**

(30) Priorität: **23.05.79 DE 2920975**

(43) Veröffentlichungstag der Anmeldung: **10.12.80**
**Patentblatt 80/25**

(84) Benannte Vertragsstaaten: **AT BE CH DE FR GB IT LI NL SE**

(71) Anmelder: **SIEMENS AKTIENGESELLSCHAFT Berlin und München, Postfach 22 02 61, D-8000 München 22 (DE)**

(72) Erfinder: **Franetzki, Manfred, Dr.-Ing., Schleifweg 7 b, D-8521 Uttenreuth (DE)**
Erfinder: **Prestele, Karl, Dipl.-Phys., Bismarckstrasse 21 d, D-8520 Erlangen (DE)**

(54) **Extrakorporal tragbares Infusionsgerät.**

(57) Die Erfindung bezieht sich auf ein extrakorporal tragbares Infusionsgerät zur dosierbaren Abgabe von Flüssigkeiten, bestehend aus einem Gerätegehäuse mit einer Förder- und Dosiereinheit zum Fördern der Flüssigkeit aus einem Vorratsbehälter zur Ausflussöffnung eines in den Patientenkörper einsetzbaren Katheters und zugehöriger Antriebs- und Steuervorrichtung. Bei einem bekannten Gerät dieser Art muss eine Kolbenspitze mit Vorratsbehälter täglich ausgetauscht werden; das Gerät ist also nicht langzeitbetreuungsfrei. Gemäss der Erfindung wirkt eine Pumpe (13) als Förder- und Dosiereinheit in an sich bekannter Weise lediglich auf das jeweils zu fördernde Flüssigkeitsvolumen in einem zwischen Vorratsbehälter (12) und Katheter (16) angeordnetem Förderschlauch (14) ein, wobei aber speziell Flüssigkeitsvorratsbehälter (12, 20, 24), Förderschlauch (14) und Katheter (16) Austauschteile und derart im Gerätegehäuse (6) angeordnet sind, dass sie jederzeit zum Ersatz von Infusionsflüssigkeit und/oder zum Legen eines neuen Katheters (16) trennbar und nach Austausch wieder an den Anschlussstellen betriebsmässig miteinander verbindbar sind. Das erfindungsgemässe Gerät ist besonders applikationsfreundlich.

EP 0 019 817 A1

ACTORUM AG

SIEMENS AKTIENGESELLSCHAFT     Unser Zeichen
Berlin und München            VPA 79 P 5034 EUR

Extrakorporal tragbares Infusionsgerät

Die Erfindung bezieht sich auf ein extrakorporal tragbares Infusionsgerät zur dosierbaren Abgabe von Flüssigkeiten, bestehend aus einem Gerätegehäuse mit einer Förder- und Dosiereinheit zum Fördern der Flüssigkeit aus einem Vorratsbehälter zur Ausflußöffnung eines in den Patientenkörper einsetzbaren Katheters und zugehöriger Antriebs- und Steuervorrichtung.

Bei der Behandlung von Patienten mit flüssigen Medikamenten ist eine fest eingestellte Rate der Infusionsflüssigkeit häufig unzureichend und es soll daher möglich sein, die pro Zeiteinheit infundierte Flüssigkeitsmenge in bestimmten Zeitabständen nachzuregeln, umzusteuern oder auch programmäßig zu verändern. Dies ist insbesondere dann erforderlich, wenn zur Diabetes-Therapie Insulin fortwährend infundiert werden soll, weil der Insulinbedarf des Zuckerkranken während des Tages, bedingt z. B. durch den Rhythmus der Mahlzeiten,

Wht 5 Rl / 17.5.1979

großen Schwankungen unterworfen ist, während er hingegen während der Nacht zeitlich nahezu konstant ist.

Es sind implantierfähige Infusionsgeräte bekannt geworden, die obige Forderungen erfüllen. Da derartige Geräte über längere Zeiten im Körper getragen werden sollen, müssen hinsichtlich Betriebszuverlässigkeit, Programmübertragung sowie hinsichtlich Gewebeverträglichkeit der verwendeten Materialien besonders hohe Anforderungen gestellt werden. Es ist daher auch vorgeschlagen worden, sich für bestimmte Anwendungszwecke mit extrakorporal am Patientenkörper tragbaren Geräten zu behelfen. Viele der bei implantierfähigen Geräten äußerst kritischen Forderungen fallen dann weg. Beispielsweise Medikamentenlagerung einerseits und Energiespeicherung für den Antrieb andererseits sind dann wesentlich unproblematischer. Speziell für solche Patienten, die nur eine vorübergehende Behandlung benötigen, können daher extrakorporal tragbare Infusionsgeräte vorteilhaft angewendet werden.

Aus der DE-OS 28 09 990 ist ein Gerät der eingangs genannten Art bekannt. Dieses Gerät besteht im wesentlichen aus einer motorgetriebenen Injektionsspritze als Förder- und Dosiereinheit, die in einem Gehäuse am Arm des Patienten tragbar ist. Zwecks Ersatz von Infusionsflüssigkeit muß bei diesem Gerät jeweils die gesamte Injektionsspritze als Förder- und Dosiereinheit ausgetauscht werden.

Das Gerät nach der DE-OS 28 09 990 hat zwar den Vorteil der schnellen Austauschbarkeit der Spritze; derartige Spritzen haben jedoch durch den Spritzenkolben einen großen Raumbedarf im Gerätegehäuse und ein vergleichsweise geringes Speichervolumen; sie müssen daher häufig ausgewechselt werden. Das bekannte Gerät ist also nicht über einen längeren Zeitraum betreuungsfrei. Eine typische Eigenart der bei solchen Infusionsgeräten verwendeten Spritzen als Förder- und Dosiereinheit ist weiterhin, daß der Antrieb jeweils auf das gesamte Vorratsvolumen einwirkt. Wegen der offenen Verbindung von Vorratsbehälter und Katheter kann es bei derartigen Geräten nicht ausgeschlossen werden, daß durch mechanische Einwirkungen auf Spritze oder Spritzenkolben, z. B. infolge eines Unfalls, der Patient durch eine Überdosis des Medikaments gefährdet wird. Außerdem ist es auch schwierig, die für den bestimmungsgemäßen Zweck benötigten kleinsten Dosierungen mit solchen automatisierten Spritzen zuverlässig erreichen.

Aufgabe der Erfindung ist daher, ein Infusionsgerät zu schaffen, das vom Patienten ständig am Körper getragen werden kann, einen Zugriff zum Gerät durch den Arzt o. dgl. ermöglicht, aber auch vom Patienten selbständig hinsichtlich Änderung der Infusionsparameter bedienbar ist. Das Infusionsgerät soll also zumindest über einige Wochen betreuungsfrei betrieben werden können, so daß es für eine ambulante Anwendung am Patienten geeignet ist.

Die Aufgabe ist erfindungsgemäß dadurch gelöst, daß eine Pumpe als Förder- und Dosiereinheit in an sich bekannter Weise lediglich auf das jeweils zu infundierende Flüssigkeitsvolumen in einem zwischen Vorratsbehälter und Katheter angeordentem Förderschlauch einwirkt, wobei aber speziell Flüssigkeitsvorratsbehälter, Förderschlauch und Katheter Austauschteile sind, die jederzeit zum Ersatz von Vorratsflüssigkeit und/oder zum Legen eines neuen Katheters trennbar und nach Austausch wieder betriebsmäßig miteinander verbindbar sind.

Es ist somit also ein Infusionsgerät geschaffen, das einerseits den hohen, normalerweise nur an implantierfähige Infusionsgeräte gestellten Sicherheitsanforderungen genügt und gegen gefährliche Überdosierungen sicher ist, das aber andererseits bei Bedarf den Austausch von Vorratsbehälter, Förderschlauch und/oder Katheter jederzeit ermöglicht und bei kleinen äußeren Abmessungen ein großes Volumen für den Vorratsbehälter ermöglicht. Ein solches Gerät kann über längere Zeiträume ambulant getragen werden.

Der Vorratsbehälter für die Flüssigkeit ist vorzugsweise aus einem flexiblen, biegeschlaffen Material gebildet, so daß der Behälter bei Entleeren der Flüssigkeit in sich zusammenfällt. Dadurch wird gewährleistet, daß der gesamte Flüssigkeitsvorrat des Vorratsbehälters über den Förderschlauch zur Austrittsstelle gefördert wird. Vorzugsweise weist der Vorratsbehälter eine Anschlußstelle für den Förderschlauch auf, mit der ein Anschlußteil des Förderschlauches fest verbunden ist, wobei die Verbindung von Förderschlauch und Katheter lösbar ist. Der Vorratsbehälter kann aber auch an der Anschlußstelle eine Materialverdickung aus elastischem, selbstschließendem Material aufweisen, in die eine mit dem distalen Ende des Förderschlauches verbundene Einstichkanüle zum Anschluß eingestochen wird. Dabei kann das proximale Ende des Förderschlauches fest mit Katheter verbunden sein.

Im Rahmen der Erfindung ist es auch möglich, starr ausgebildete Vorratsbehälter zu verwenden. In diesem Fall sind zusätzliche Mittel für den Vorratsbehälter vorgesehen, die das vollständige Entleeren der Flüssigkeit des Vorratsbehälters gewährleisten.

Als Pumpe wird in vorteilhafter Ausgestaltung eine Rollenpumpe mit wenigstens zwei Pumpenrollen verwendet. Solche Rollenpumpen können mit frei tragenden oder in Abrollbacken angeordneten Förderschläuchen ausgebildet sein. Der Abrollbacken kann zum Einlegen des Förderschlauches manuell verschiebbar oder verschwenkbar angeordnet sein. Bei solchen Pumpenanordnungen kann also der Förderschlauch leicht ausgetauscht werden.

Insgesamt ergibt sich also ein äußerst kompaktes Infusionsgerät, das vom Patienten über längere Zeit ohne Beeinträchtigung tragbar ist. Mit der Antriebs- und Steuereinrichtung für die Rollenpumpe kann die Infusion entsprechend dem Tagesbedarf gesteuert werden. Dabei wird in vorteilhafter Weise einer konstanten Infusionsrate als Basalrate bei Bedarf eine zusätzliche Infusionsdosis als Aufsetzprogramm zugeschaltet, die vom Patienten nach Mahlzeiten o. dgl. direkt abrufbar ist.

Weitere Einzelheiten und Vorteile der Erfindung ergeben sich aus nachfolgender Figurenbeschreibung von Ausführungsbeispielen anhand der Zeichnung.

Es zeigen:

Fig. 1 schematisch einen Patienten mit einem extern am Körper angeordneten Infusionsgerät,

Fig. 2 perspektivisch das geöffnete Infusionsgerät nach der Erfindung im teilweise aufgeschnittenen Zustand,

- 6 -   VPA 79 P 5034 EUR

Fig. 3 perspektivisch einen ersten Flüssigkeitsvorratsbehälter mit festangeschlossenem
Förderschlauch und damit verbindbaren
Ausflußkatheter,

Fig. 4 eine zu Fig. 3 alternative Anschlußmöglichkeit für einen Förderschlauch,

Fig. 5 und 6 im Schnitt zwei weitere, alternativ
zu Fig. 3 zu verwendende Vorratsbehälter mit austauschbarem Förderschlauch und Katheter und

Fig. 7 bis 9 die Aufsicht auf geöffnete erfindungsgemäße Infusionsgeräte mit
unterschiedlich ausgebildeten Rollenpumpen als Förder- und Dosiereinheit.

In den Figuren sind identische Teile mit den gleichen
Bezugszeichen versehen.

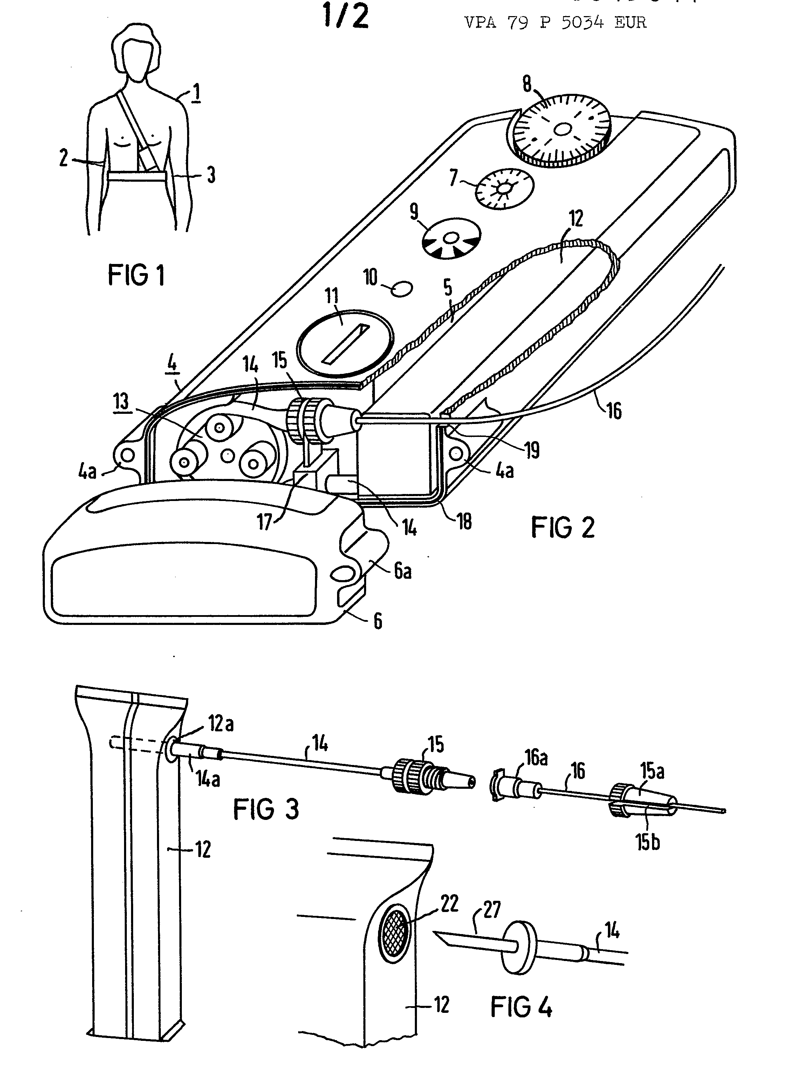

Die Fig. 1 zeigt einen Patienten 1, der an einem halfterartigen Tragegurt 2 etwa in Taillenhöhe extrakorporal
ein Infusionsgerät 3 trägt. Von diesem externen Infusionsgerät 3 ist ein (in Fig. 1 nicht sichtbarer)
Katheter entweder subkutan, intraperitoneal oder in
eine Thoraxvene gelegt. In alternativer Ausbildung
kann das Infusionsgerät 3 auch am Oberarm getragen
werden. Dazu wird das Gerät 3 mit zwei parallelen
Gurten am Arm des Patienten 1 angeschnallt und der
Katheter in eine Arm- oder Thoraxvene gelegt.

In der Fig. 2 ist mit 4 das Gehäuse des Infusionsgerätes bezeichnet, das in etwa quaderförmiger Grundform mit den Kantenmaßen von ca. 110, 70 und 25 mm ausgebildet ist. Im seitlichen Bereich ist das Gehäuse 4 aufgeschnitten dargestellt, wobei im wesentlichen zwei getrennte Teilbereiche im Gehäuseinneren erkennbar sind. Der etwa zylinderförmig ausgebildete Teilraum 5 ist durch eine Wand dicht gegenüber dem übrigen Gehäuse·4 abgeschlossen und nimmt einen Flüssigkeitsvorratsbehälter 12 auf, während der verbleibende Raum des Gehäuses 4 eine Rollenpumpe 13 mit Förderschlauch 14 als Förder- und Dosiereinheit mit zugehörigem Antrieb und Steuerelektronik sowie eine Batterie zur Energieversorgung beinhaltet. Weiterhin dargestellt ist eine Haube 6, mit der das Gerätegehäuse 4 für den betriebsmäßigen Gebrauch abdeckbar und an Verbindungsnocken 4a und 6a verriegelbar ist. In die Gehäusekante ist eine elastische Dichtung 18 eingelassen, so daß das mit der Haube 4 abgedeckte und verriegelte Gehäuse 4 abgedichtet wird. Dadurch ist das Innere des Infusionsgerätes einerseits vor Verschmutzung und Feuchtigkeit geschützt; andererseits wird so eine Flüssigkeitsverdunstung aus Vorratsbehälter 12 und Förderschlauch 14 weitgehend verhindert.

Am Gerätegehäuse 4 sind außen zwei Wahlscheiben 7 und 8 zur Einstellung der Infusionsparameter angeordnet. Die Einstellung der ersten Wahlscheibe 7 ist nur mit mechanischen Hilfsmitteln, z. B. einem Schraubenzieher, möglich. Mit dieser ersten Wahlscheibe 7 ist eine Basalrate der Infusion einstellbar. Mit der zweiten Wahlscheibe 8 können bei Bedarf zusätzlich zur konstant eingestellten Basalrate definierte Dosismengen von Infusionsflüssigkeit abgerufen werden, die nach eingespeichertem Programm innerhalb von ein oder zwei Stunden abgegeben werden. Daher ist diese Wahlscheibe 8 vom Patienten mit Hand bedienbar. Am Gerätegehäuse 4 ist der Wahlscheibe 8 ein Programmwahlschalter 9 zugeordnet, mit dem bei vorgegebener Dosismenge die alternative Variation von Infusionsrate oder Infusionszeit als unterschiedliche Programmarten eingestellt werden kann. Der Programmschalter 9 ist als Wahlscheibe ebenfalls so ausgebildet, daß die Programmeinstellung wiederum nur mit einem mechanischen Hilfsmittel möglich ist. Mittels Leuchtdiode 10 wird die Funktion der Förder- und Dosiereinheit angezeigt.

Die spezielle Ausbildung der drei Wahlscheiben 7, 8 und 9 hat den Sinn, daß Basalrate der Infusion und Programmart vom Arzt festgelegt und eingestellt werden und vom Patienten bei ambulanter Behandlung im allgemeinen nicht verändert werden sollen. Der Patient wählt lediglich als einzige Kenngröße zum geeigneten Zeitpunkt, etwa nach einer Mahlzeit entsprechend der aufgenommenen Nahrungsmenge die notwendige Zusatzinfusionsdosis an, womit das Aufsetzprogramm auf die Basalrate gestartet wird. Ein solches Aufsetzprogramm ist rechteckförmig entweder mit konstanter Aufsetzzeit und variabler Infusionsrate oder mit konstanter Infusionsrate und variabler Aufsetzzeit ausgebildet. Im ersten Fall hat sich eine Zeit von 1 h und Infusionsraten in Schritten von 1 IE/h zwischen 1 bis 10 IE/h, im zweiten Fall eine Infusionsrate von 6 IE/h* und Zeitschritte von 10 min zwischen 10 und 100 min als geeignet erwiesen. *(Internationale (Insulin)-Einheiten pro Stunde) Am Gerätegehäuse befindet sich weiterhin eine lösbare Verschlußklappe 11, die den Zugang zur Energieversorgungseinheit (Batterie) schafft. Zur Energieversorgung des Infusionsgerätes werden handelsübliche Batterien verwendet, die lange Lebensdauer aufweisen und schnell austauschbar sind. Ein Austausch der Batterien erfolgt jeweils bei Austausch des Vorratsbehälters 12.

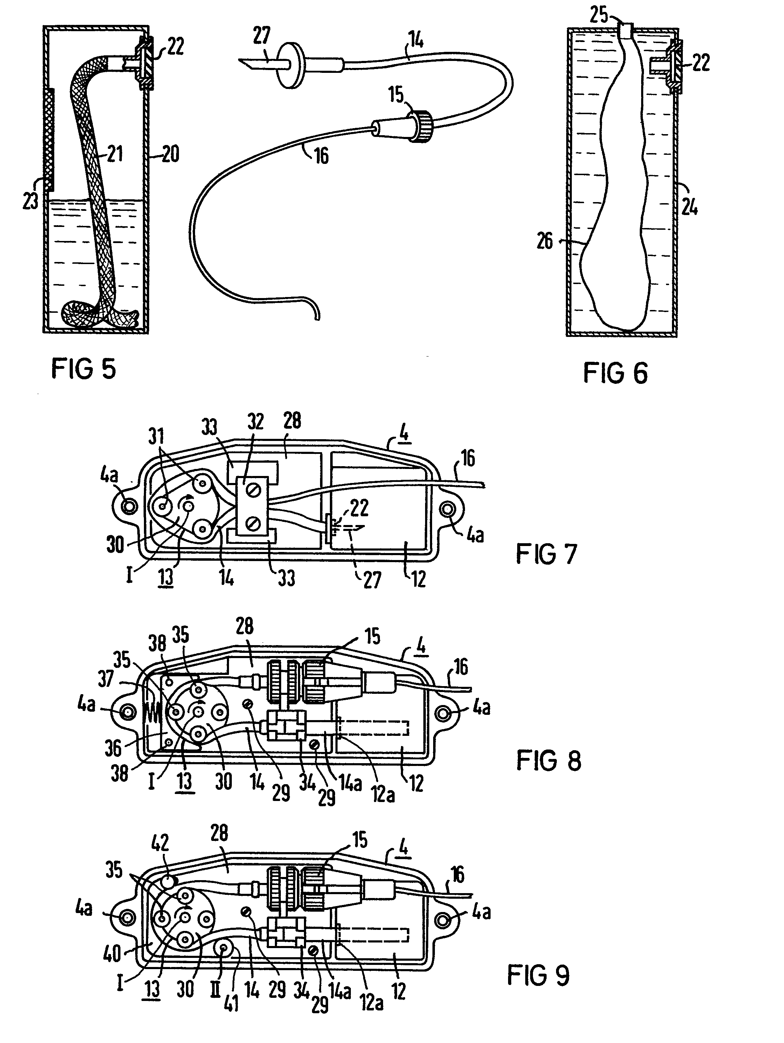

Im seitlichen Teil des Gerätegehäuses 4 ist im Teilraum 5 des Vorratsbehälters 12 als länglicher, etwa
prismenförmiger Körper angedeutet. Mit dem Vorratsbehälter 12 ist der Pumpenkopf 13 einer Rollenpumpe als
Förder- und Dosiereinheit über einen Förderschlauch 14
betriebsmäßig verbunden. Der Pumpenkopf 13 ist in Fig. 2
lediglich schematisch angedeutet und wird in Fig. 7 bis
9 in unterschiedlicher Ausbildung im einzelnen beschrieben. Der Förderschlauch 14 wird an seinen Enden
durch eine Halterung 17 in der richtigen Stellung
fixiert. Am proximalen Ende des Förderschlauches 14
ist über eine lösbare Verbindung 15 ein Katheter 16
angeschlossen, der in geeigneter Weise in den Patientenkörper gelegt werden kann. Das Gehäuse 4 weist zur betriebssicheren Durchführung des Katheters 16 eine Ausnehmung 19 auf, die auch die Dichtheit des Innenraums
gewährleistet.

In der Fig. 3 ist der Vorratsbehälter 12 als flexibler
Kunststoffbalg dargestellt, der eine etwa quadratische
Grundfläche aufweist und an seinem oberen Ende als
spitze Tüte ausläuft. Eine solche Raumform hat sich
bei der Serienherstellung als geeignet erwiesen. Der
zugehörige Förderschlauch 14 hat an seinem distalen
Ende 14a ein entsprechendes Anschlußrohr, das an einer
ringförmigen Verdickung fest mit dem Behälter 12 verschweißt ist und als Stutzen in den Behälter hinein
führt. Das proximale Ende des Förderschlauches 14 ist
über einen lösbaren Anschluß 15 dicht mit dem Katheter
16 verbunden. Dabei ist das Anschlußteil 15 mit Luer-
Konus fest mit dem Förderschlauch 14 verbunden, auf
den ein mit dem Katheter 16 verbundenes Gegenstück 16a
aufgesteckt und mittels Überwurfmutter 15a fixiert
wird. Die Überwurfmutter 15a ist seitlich geschlitzt,
so daß sie auf einen gelegten Katheter durch den Schlitz
15b seitlich aufgeschoben werden kann.

- 10 -     VPA 79 P 5034 EUR

In der Fig. 4 ist in einem Teilausschnitt der Vorratsbehälter 12 mit einer Anschlußstelle 22, aus Naturgummi dargestellt, in die eine mit dem distalen Ende
des Förderschlauches 14 fest verbundene Einstichkanüle
27 eingestochen werden kann. Bei geeigneter Materialverdickung dichtet die Gummianschlußstelle 22 die Einstichsöffnung nach Anschluß des Förderschlauches jeweils selbsttätig ab. Eine solche Anschlußmöglichkeit
ist alternativ zu der anhand Fig. 3 beschriebenen
festen Verbindung zu verwenden.

Für den bestimmungsmäßigen Gebrauch ist es wichtig,
daß Vorratsbehälter 12, Förderschlauch 14 und Katheter
16 jederzeit wenigstens an einem Punkt trennbar und
wieder betriebsmäßig miteinander verbunden werden
können. Dies ist z. B. dann notwendig, wenn bei Gebrauch der Infusionsgeräte lediglich ein Katheter
neu gelegt werden muß, um Infektionen zu verhindern,
wobei Gerätegehäuse 4 mit Vorratsbehälter 12 und
Förderschlauch 14 in Funktionsstellung bleiben. In
anderen Fällen soll dagegen während der Behandlung
nur der geleerte Vorratsbehälter 12 zum Ersatz von
Infusionsflüssigkeit ausgetauscht werden, wobei der
Katheter 16 gesetzt bleiben kann.

Der Vorratsbehälter 12 in Fig. 3 und 4 ist aus flexiblem, biegeschlaffen Material gebildet, so daß er
nach Entleeren der Flüssigkeit nachgibt und in sich
zusammenfällt. Entscheidende Bedingung für die Materialwahl ist dabei eine zu vernachlässigende Gas-
und Wasserdampfdurchlässigkeit. Geht man davon aus,
daß im Vorratsbehälter Infusionsflüssigkeit für etwa
30 Tage vorhanden ist, so darf während dieser Einsatzzeit keine nennenswerte Diffusion von Luft in den
Behälter bzw. Flüssigkeitsdampf aus dem Behälter erfolgen. Unter diesen Voraussetzungen ist an der Anschlußstelle 12a für den Förderschlauch 14 Flüssigkeit mit definierter Konzentration bis zur vollständigen Entleerung des Vorratsbehälters 12 vorhanden. Als Materialien für derartige Behälter haben sich sog. Verbundfolien bewährt, die beispielsweise auf Polyäthylen/Polyamid- oder Polyäthylen/Polyacrylnitrilbasis bekannt sind. Dieses Material hat insbesondere den Vorteil der gleichzeitigen Wasserdampf- und Luftundurchlässigkeit und ist dabei besonders als Speicher für Insulin in wäßriger Lösung geeignet. Die Wandstärke eines aus solchem Material hergestellten Behälters beträgt dabei etwa 100 µm.

In der Fig. 5 und 6 sind zwei weitere, alternativ zu verwendende Vorratsbehälter 20 und 24 dargestellt, die starre Wände aufweisen. Dabei ist in den ersten Behälter 20 von einer Anschlußstelle 22 mit Einstichgummi und Rohransatz für eine Einstichkanüle ein hydrophiler Docht 21 in dessen Inneres weiträumig hineingeführt, der in Kontakt mit der Flüssigkeit steht und so zum Nachtransport der Flüssigkeit sorgt. Über einen hydrophoben Keimfilter 23 aus Teflon kann bei Entleeren des Behälters Luft eindringen und das Volumen der geförderten Flüssigkeit ausfüllen. Der gleiche Zweck wird bei dem zweiten starren Behälter 24 dadurch erreicht, daß ein unmittelbar über einen Anschluß 25 mit Umgebungsluft verbundener flexibler Behälter 26 den nach Entleeren der Flüssigkeit frei werdenden Raum einnimmt. An den Anschlußstellen 22 wird demnach immer Flüssigkeit bis zur vollständigen Entleerung der Vorratsbehälter 20 und 24 vorhanden sein.

In den Fig. 7 bis 9 ist im Umriß jeweils die obere Grundfläche des Gerätegehäuses 4 mit Verschlußnocken 4a angedeutet. Es sind im einzelnen drei alternativ zu verwendende Typen von Rollenpumpen dargestellt. Gemeinsam ist den drei Figuren die gleiche Anordnung von Vorratsbehälter 12 und Grundplatte 28 und Drehscheibe 30 des Pumpenkopfes 13.

Alle drei Pumpentypen sind derart aufgebaut, daß ein Austausch von Vorratsbehälter 12, Förderschlauch 14 und Katheter 16 schnell und komplikationslos durchführbar ist. Die einzelnen Pumpenrollen sind derart in die Drehscheibe 30 eingelassen, daß eine Führung für den Förderschlauch gebildet wird. In den Figuren 7 bis 9 ist der obere Teil der Drehscheibe 30 jeweils weggeschnitten.

Die Fig. 7 zeigt eine Rollenpumpe mit drei Pumpenrollen 31 in Dreiecksanordnung auf einer kreisförmigen Drehscheibe 30 mit Drehachse I. Um diese Dreiecksanordnung ist der Förderschlauch 14 freitragend gelegt und mittels eines Kassettenverschlusses 32 eingespannt, der in zugehörigen Lagerelementen 33 auf der Grundplatte 28 fixierbar ist. Derartige freitragende Rollenpumpen sind äußerst einfach aufgebaut. Sie stellen jedoch hinsichtlich Reproduzierbarkeit der Förderraten bestimmte Anforderungen an den Förderschlauch und dessen Vorspannung beim Einspannen in die Kassette 32. Zweckmäßigerweise wird bei der Herstellung Förderschlauch 14, Katheter 16 und Kassettenverschluß/als ein Fertigteil vorgefertigt, so daß eine Justierung vom Benutzer nicht mehr notwendig ist.

In der Fig. 8 sind vier Pumpenrollen 35 symmetrisch am Umfang der kreisförmigen Drehscheibe 30 mit Drehachse I angeordnet. Der mit dem Vorratsbehälter 12 fest verbundene Förderschlauch 14 ist beidseitig des Pumpenkopfes 13 durch eine Halterung 34 fixiert. Im Pumpenkopfbereich verläuft der Förderschlauch 14 auf der inneren Ausnehmung eines Abrollbackens 36 als Gegenlager auf der Grundplatte 28. Dabei ist der Abrollbacken 36 mittels Federeinrichtung 37 in einer Parallelführung 38 verschiebbar . Zum Austausch des Förderschlauches 14 wird der Schlauchträger mit einer Handhabe 39 in der Führung 36 verschoben.

In der Fig. 9 ist die Rollenpumpe nach Fig. 8 in der Weise modifiziert, daß auf der Grundplatte 28 ein Abrollbacken 40 schwenkbar an einem Schwenklager 41 mit Drehachse II angelenkt ist. Dabei kann der Förderschlauch 14 bei ausgeschwenktem Schlauchträger 40 in einfacher Weise um die Pumpenrollen 35 gelegt werden und dann nach Zurückschwenken des Schlauchträgers 40 und Einklinken durch einen Rasterstift 42 fixiert werden.

Bei den Rollenpumpen nach Fig. 8 und 9 ist es erforderlich, daß der Abrollbacken 36 bzw. 40 gegenüber der Drehscheibe 30, die ortsfest im Gehäuse 4 angeordnet ist, justiert werden kann. Aus diesem Grunde ist die Grundplatte 28, auf der die Abrollbacken 36 und 40 gelagert sind, mittels Schrauben 29 justierbar auf dem Gehäuse 4 befestigt.

Die Rollenpumpen nach Fig. 7 bis 9 sind also jeweils so ausgebildet, daß mit wenigen Handgriffen der Förderschlauch 14 ersetzt werden kann. Dabei ist die Trennstelle Vorratsbehälter 12, Förderschlauch 14 und Katheter 16 entweder unmittelbar am Vorratsbehälter mit fester Verbindung des Katheters 16 oder zwischen Förderschlauch 14 und Katheter 16 mit fester Verbindung zum Vorratsbehälter 12 angeordnet.

Anhand der Figuren wurden Ausführungsbeispiele der Erfindung in verschiedenen Weiterbildungen beschrieben. Diese einzelnen Varianten sind natürlich auch in anderer Kombination erfindungsgemäß aufbaubar.

Die Erfindung wurde speziell in Verwendung als Insulininfusionsgerät für die Diabetes-Therapie beschrieben. Auch andere flüssige Medikamente sind mit einem erfindungsgemäßen Infusionsgerät dosiert abgebbar.

Patentansprüche

1. Extrakorporal tragbares Infusionsgerät zur dosierbaren Abgabe von Flüssigkeiten, bestehend aus einem Gerätegehäuse mit Förder- und Dosiereinheit zum Fördern der Flüssigkeit aus einem Vorratsbehälter zur Ausflußöffnung eines in den Patientenkörper einsetzbaren Katheters und zugehöriger Antriebs- und Steuervorrichtung, d a d u r c h  g e k e n n z e i c h - n e t , daß eine Pumpe (13) als Förder- und Dosiereinheit in an sich bekannter Weise lediglich auf das jeweils zu infundierende Flüssigkeitsvolumen in einem zwischen Vorratsbehälter (12) und Katheter (16) angeordneten Förderschlauch (14) einwirkt, wobei aber speziell Flüssigkeitsvorratsbehälter (12), Förderschlauch (14) und Katheter (16) Austauschteile und derart im Gerätegehäuse (4) angeordnet sind, daß sie jederzeit zum Ersatz von Infusionsflüssigkeit und/oder zum Legen eines neuen Katheters (16) trennbar und nach Austausch wieder an den Anschlußstellen (15, 16a, 22, 27) betriebsmäßig miteinander verbindbar sind.

2. Infusionsgerät nach Anspruch 1, d a d u r c h  g e k e n n z e i c h n e t , daß der Vorratsbehälter (12) aus einem flexiblen, biegesdhlaffen Material gebildet ist, so daß der austauschbare Vorratsbehälter (12) bei Entleeren der Flüssigkeit in sich zusammenfällt, wobei das Material eine Verbundfolie auf Polyäthylen/Polyamid- oder Polyäthylen/Polyacrylnitril-Basis mit einer Wandstärke zwischen 50 und 200 $\mu$m ist, die eine zu vernachlässigende Gasdurchlässigkeit, insbesondere für Wasserdampf einerseits und Luft andererseits, hat.

3. Infusionsgerät nach Anspruch 1, d a d u r c h  g e k e n n z e i c h n e t , daß der austauschbare

Wht 5 Rl / 17.4.1980

Vorratsbehälter ein starrer Behälter (20, 24) ist, dem Mittel (23, 25, 26) zum Ersatz des Volumens der geförderten Flüssigkeit zugeordnet sind, wobei das geförderte Flüssigkeitsvolumen entweder durch Luft, die über einen hydrophoben Filter (23) in den Vorratsbehälter (20) eintreten kann, oder durch einen flexiblen, sich über einen Anschluß (25) mit Luft füllenden Behälter (26) innerhalb des Vorratsbehälters (24) ausgefüllt wird.

4. Infusionsgerät nach Anspruch 1, d a d u r c h  g e k e n n z e i c h n e t , daß der austauschbare Vorratsbehälter (12) mit dem distalen Ende des Förderschlauches (14) fest verbunden ist, wobei der Förderschlauch (14) an seinem proximalen Ende einen lösbaren Anschluß (15, 15a, 16a) für den Katheter (16) aufweist.

5. Infusionsgerät nach Anspruch 1, d a d u r c h  g e k e n n z e i c h n e t , daß der Vorratsbehälter (12, 20, 24) eine Anschlußstelle (12a) für das distale Ende des Förderschlauches (14) aus einer Materialverdickung (22) aus selbstschließenden, elastischen Material, beispielsweise Naturgummi, aufweist, wobei der Förderschlauch (14) an seinem distalen Ende eine Einstichkanüle (27) zum Einstechen in die Materialverdickung (22) hat.

6. Infusionsgerät nach Anspruch 4 oder 5, d a - d u r c h  g e k e n n z e i c h n e t , daß Förderschlauch (14) und Katheter (16) durch einen Luer-Verschluß (15) verbunden sind, der durch eine Überwurfmutter (15a), die zum nachträglichen Aufschieben über den Katheter (16) seitlich geschlitzt ist, abdichtbar und fixierbar ist.

7. Infusionsgerät nach Anspruch 1, d a d u r c h g e k e n n z e i c h n e t , daß die Pumpe (13) eine Rollenpumpe mit wenigstens zwei Pumpenrollen (31) ist, die den Förderschlauch (14) frei tragen, wobei in Arbeitsstellung der Rollenpumpe (13) der Förderschlauch (14) durch eine Spannkassette (33) für den Förderschlauch (14), die in einem Gegenlager (32) einklinkbar ist, vorgespannt gehalten wird.

8. Infusionsgerät nach Anspruch 1 und 12, d a - d u r c h g e k e n n z e i c h n e t , daß die Pumpe eine Rollenpumpe mit wenigstens zwei Pumpenrollen (35) und einem Abrollbacken (36, 40) als Gegenlager zum Abquetschen des Förderschlauches (14) ist, wobei der Abrollbacken (36, 40) auf einer Grundplatte (18) gegenüber dem im Gehäuse (4) ortsfesten Pumpenantrieb justierbar und entweder zum Austausch des Förderschlauches (14) mittels einer Federhalterung (37) elastisch in Parallelführungen (38) verschiebbar oder zum Austausch des Förderschlauches (14) in einem Lager (41) schwenkbar angelenkt ist, wobei der Backen (40) durch eine Raste (42) in Arbeitsstellung fixierbar ist.

9. Infusionsgerät nach Anspruch 1, d a d u r c h g e k e n n z e i c h n e t , daß durch die Steuer- vorrichtung einer wählbaren, zeitlichen konstanten Infusionsrate als Basalrate eine wählbare zusätzliche Infusionsdosis als zeitlich oder infusionsratenmäßig veränderbares Aufsetzprogramm zugeschaltet werden kann, wobei das Aufsetzprogramm rechteckförmig entweder mit konstanter Aufsetzzeit und variabler Infusionsrate oder mit konstanter Infusionsrate und variablen Zeiten veränderbar ist.

10. Infusionsgerät nach Anspruch 1, d a d u r c h . g e k e n n z e i c h n e t , daß am Gerätegehäuse (4) Bedienungselemente (7, 8) für vom Patienten einstellbare Basalrate und zuschaltbare Infusionsdosis angeordnet sind und daß am Gerätegehäuse (4) ein Umschalter (9) zur Einstellung verschiedener programmäßig vorbestimmter Aufsetzprogramme vorhanden ist.

11. Infusionsgerät nach Anspruch 1, d a d u r c h g e k e n n z e i c h n e t , daß am Gerätegehäuse (4) Mittel (10) zur Anzeige der Gerätefunktionen angeordnet sind.

0019817
VPA 79 P 5034 EUR

FIG 1

FIG 2

FIG 3

FIG 4

0019817

VPA 79 P 5034 EUR

FIG 5

FIG 6

FIG 7

FIG 8

FIG 9

Europäisches
Patentamt

**EUROPÄISCHER RECHERCHENBERICHT**

00198 17

Nummer der Anmeldung

EP 80 10 2735

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | MEDICAL PROGRESS THROUGH TECHNO-LOGY, Band 5, Nr. 4, Mai 1978 Springer-Verlag (DE) A.M. ALBISSER et al.: "A Portable precision pumping system for chronic, programmed insulin in-fusion", Seiten 187-193. <br><br> * Seite 188, Spalte 1, Absätze 2,3; Spalte 2, Absatz 1; Seite 190, Spalte 1, Absätze 1-3 * | 1,2,4, 6,7 |
| X | DE - A - 1 491 848 (UNITED STATES CATHETER & INSTRUMENT CORP.) <br><br> * Abbildungen 3,7 und 11; Seite 7, Absatz 3; Seite 8; Seite 9, Absätze 3 und 4; Seite 10; Seite 11, Absatz 3 * | 1,3,4, 5,6,8 |

**KLASSIFIKATION DER ANMELDUNG (Int. Cl.³)**

A 61 M 5/14

**RECHERCHIERTE SACHGEBIETE (Int. Cl.³)**

A 61 M
A 61 F
F 04 B

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patent-familie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 03-09-1980 | NOESEN |

EPA form 1503.1  06.78